# EUROPEAN PATENT SPECIFICATION

(11) **EP 1 330 526 B1**
(45) Date of publication and mention of the grant of the patent: **21.04.2010**
(21) Application number: 01992788.8
(22) Date of filing: 28.09.2001
(51) Int. Cl.: C12N 15/53

(54) **PROCESS FOR THE FERMENTATIVE PREPARATION OF L-AMINO ACIDS USING STRAINS OF THE ENTEROBACTERIACEAE FAMILY**
VERFAHREN ZUR FERMENTATIVEN HERSTELLUNG VON L-AMINOSÄUREN UNTER VERWENDUNG VON STÄMMEN AUS DER FAMILIE DER ENTEROBACTERIACEAE
PROCEDE DE PREPARATION PAR FERMENTATION D'ACIDES L-AMINES AU MOYEN DE SOUCHES DE LA FAMILLE DES ENTEROBACTERIES

(30) Priority: 04.11.2000 DE 10054748; 15.11.2000 US 248210 P; 14.03.2001 DE 10112107; 16.04.2001 US 283612 P
(43) Date of publication of application: 30.07.2003
(73) Proprietor: Evonik Degussa GmbH, 45128 Essen (DE)
(72) Inventor: RIEPING, Mechthild, 33619 Bielefeld (DE); THIERBACH, Georg, 33613 Bielefeld (DE)
(86) International application number: PCT/EP2001/011228
(87) International publication number: WO 2002/036797

(56) References cited:
- EP-A- 1 096 013
- WO-A-01/71012
- US-A- 4 278 765
- DATABASE BIOSIS [Online] BIOSCIENCES INFORMATION SERVICE, PHILADELPHIA, PA, US; 1989 GRABAU C ET AL: "LIPID BINDING BY ESCHERICHIA-COLI PYRUVATE OXIDASE IS DISRUPTED BY SMALL ALTERATIONS OF THE CARBOXYL-TERMINAL REGION" Database accession no. PREV198988088849 XP002208064 & JOURNAL OF BIOLOGICAL CHEMISTRY, vol. 264, no. 21, 1989, pages 12510-12519, ISSN: 0021-9258
- CHANG Y-Y ET AL: "MOLECULAR CLONING, DNA SEQUENCING, AND ENZYMATIC ANALYSES OF TWO ESCHERICHIA COLI PYRUVATE OXIDASE MUTANTS DEFECTIVE IN ACTIVATION BY LIPIDS" JOURNAL OF BACTERIOLOGY, WASHINGTON, DC, US, vol. 167, no. 1, July 1986 (1986-07), pages 312-318, XP000987283 ISSN: 0021-9193
- GRABAU C ET AL: "MOLECULAR CLONING OF THE GENE (POXB) ENCODING THE PYRUVATE OXIDASE OF ESCHERICHIA COLI, A LIPID-ACTIVATED ENZYME" JOURNAL OF BACTERIOLOGY, WASHINGTON, DC, US, vol. 160, no. 3, December 1984 (1984-12), pages 1088-1092, XP000987282 ISSN: 0021-9193
- DATABASE BIOSIS [Online] BIOSCIENCES INFORMATION SERVICE, PHILADELPHIA, PA, US; 1987 VAN DYK T K ET AL: "PLEIOTROPIC EFFECTS OF POX-A REGULATORY MUTATIONS OF ESCHERICHIA-COLI AND SALMONELLA-TYPHIMURIUM MUTATIONS CONFERRING SULFOMETURON METHYL AND ALPHA KETOBUTYRATE HYPERSENSITIVITY" Database accession no. PREV198784118407 XP002208065 & JOURNAL OF BACTERIOLOGY, vol. 169, no. 10, 1987, pages 4540-4546, ISSN: 0021-9193

## Description

This invention relates to a process for the fermentative preparation of L-threonine, L-lysine and L-valine, using strains of the Enterobacteriaceae family in which the poxB gene is attenuated.

### Prior Art

L-Amino acids, in particular L-threonine, L-lysine and L-valine are used in human medicine and in the pharmaceuticals industry, in the foodstuffs industry and very particularly in animal nutrition.

It is known to prepare L-amino acids by fermentation of strains of Enterobacteriaceae, in particular Escherichia coli (E. coli) and Serratia marcescens. Because of their great importance, work is constantly being undertaken to improve the preparation processes. Improvements to the process can relate to fermentation measures, such as e.g. stirring and supply of oxygen, or the composition of the nutrient media, such as e.g. the sugar concentration during the fermentation, or the working up to the product form, by e.g. ion exchange chromatography, or the intrinsic output properties of the microorganism itself.

Methods of mutagenesis, selection and mutant selection are used to improve the output properties of these microorganisms. Strains which are resistant to antimetabolites, such as e.g. the threonine analogue α-amino-β-hydroxyvaleric acid (AHV), or are auxotrophic for metabolites of regulatory importance and produce L-amino acid, such as e.g. L-threonine, are obtained in this manner.

Methods of the recombinant DNA technique have also been employed for some years for improving the strain of strains of the Enterobacteriaceae family which produce L-amino acids, by amplifying individual amino acid biosynthesis genes and investigating the effect on the production.

### Object of the Invention

The inventors had the object of providing new measures for improved fermentative preparation of L-threonine, L-lysine and L-valine.

### Description of the Invention

The invention provides a process for the fermentative preparation of L-threonine, using microorganisms of the Enterobacteriaceae family which, in particular, already produce L-threonine and in which the nucleotide sequence which codes for the enzyme pyruvate oxidase (EC 1.2.2.2) (poxB gene) is attenuated.

The term "attenuation" in this connection describes the reduction or elimination of the intracellular activity of one or more enzymes (proteins) in a microorganism which are coded by the corresponding DNA, for example by using a weak promoter or a gene or allele which codes for a corresponding enzyme with a low activity or inactivates the corresponding enzyme (protein) or gene, and optionally combining these measures.

The process comprises carrying out the following steps:
a) fermentation of microorganisms of the Enterobacteriaceae family in which at least the poxB gene is attenuated,
b) concentration of the corresponding L-amino acid in the medium or in the cells of the microorganisms of the Enterobacteriaceae family, and
c) isolation of the desired L-amino acid.

The microorganisms which the present invention provides can produce L-amino acids from glucose, sucrose, lactose, fructose, maltose, molasses, optionally starch, optionally cellulose or from glycerol and ethanol. They are representatives of the Enterobacteriaceae family chosen from the genera Escherichia, Erwinia, Providencia and Serratia. The genera Escherichia and Serratia are preferred. Of the genus Escherichia the species Escherichia coli and of the genus Serratia the species Serratia marcescens are to be mentioned in particular.

Suitable strains, which produce L-threonine in particular, of the genus Escherichia, in particular of the species Escherichia coli, are, for example
Escherichia coli TF427
Escherichia coli H4578
Escherichia coli KY10935
Escherichia coli VNIIgenetika MG442
Escherichia coli VNIIgenetika M1
Escherichia coli VNIIgenetika 472T23
Escherichia coli BKIIM B-3996
Escherichia coli kat 13
Escherichia coli KCCM-10132

Suitable L-threonine-producing strains of the genus Serratia, in particular of the species Serratia marcescens, are, for example
Serratia marcescens HNr21
Serratia marcescens TLr156
Serratia marcescens T2000.

Strains from the Enterobacteriaceae family which produce L-threonine preferably have, inter alia, one or more genetic or phenotypic features chosen from the group consisting of: resistance to α-amino-β-hydroxyvaleric acid, resistance to thialysine, resistance to ethionine, resistance to α-methylserine, resistance to diaminosuccinic acid, resistance to α-aminobutyric acid, resistance to borrelidin, resistance to rifampicin, resistance to valine analogues, such as, for example, valine hydroxamate, resistance to purine analogues, such as, for example, 6-dimethylaminopurine, a need for L-methionine, optionally a partial and compensatable need for L-isoleucine, a need for meso-diaminopimelic acid, auxotrophy in respect of threonine-containing dipeptides, resistance to L-threonine, resistance to L-homoserine, resistance to L-lysine, resistance to L-methionine, resistance to L-glutamic acid, resistance to L-aspartate, resistance to L-leucine, resistance to L-phenylalanine, resistance to L-serine, resistance to L-cysteine, resistance to L-valine, sensitivity to fluoropyruvate, defective threonine dehydrogenase, optionally a capacity for sucrose utilization, enhancement of the threonine operon, enhancement of homoserine dehydrogenase I-aspartate kinase I, preferably of the feedback-resistant form, enhancement of homoserine kinase, enhancement of threonine synthase, enhancement of aspartate kinase, optionally of the feedback-resistant form, enhancement of aspartate semialdehyde dehydrogenase, enhancement of phosphoenol pyruvate carboxylase, optionally of the feedback-resistant form, enhancement of phosphoenol pyruvate synthase, enhancement of transhydrogenase, enhancement of the RhtB gene product, enhancement of the RhtC gene product, enhancement of the YfiK gene product, enhancement of a pyruvate carboxylase, and attenuation of acetic acid formation.

It has been found that microorganisms of the Enterobacteriaceae family produce L-amino acids, in particular L-threonine, in an improved manner after attenuation, in particular elimination, of the poxB gene, which codes for pyruvate oxidase (EC number 1.2.2.2).

It has furthermore been found that microorganisms of the Enterobacteriaceae family form lower concentrations of the undesirable by-product acetic acid after attenuation, in particular elimination, of the poxB gene, which codes for pyruvate oxidase (EC number 1.2.2.2).

The nucleotide sequence of the poxB gene of Escherichia coli has been published by Grabau and Cronan (Nucleic Acids Research. 14 (13), 5449-5460 (1986)) and can also be found from the genome sequence of Escherichia coli published by Blattner et al. (Science 277, 1453 - 1462 (1997), under Accession Number AE000188. The nucleotide sequence of the poxB gene of Escherichia coli is shown in SEQ ID No. 1 and the amino acid sequence of the associated gene product is shown in SEQ ID No. 2.

The poxB genes described in the text references mentioned can be used according to the invention. Alleles of the poxB gene which result from the degeneracy of the genetic code or due to "sense mutations" of neutral function can furthermore be used.

To achieve an attenuation, for example, expression of the poxB gene or the catalytic properties of the enzyme protein can be reduced or eliminated. The two measures can optionally be combined.

The reduction in gene expression can take place by suitable culturing, by genetic modification (mutation) of the signal structures of gene expression or also by the antisense-RNA technique. Signal structures of gene expression are, for example, repressor genes, activator genes, operators, promoters, attenuators, ribosome binding sites, the start codon and terminators. The expert can find information in this respect, inter alia, for example, in Jensen and Hammer (Biotechnology and Bioengineering 58: 191-195 (1998)), in Carrier and Keasling (Biotechnology Progress 15, 58-64 (1999), Franch and Gerdes (Current Opinion in Microbiology 3, 159-164 (2000)) and in known textbooks of genetics and molecular biology, such as, for example, the textbook of Knippers ("Molekulare Genetik [Molecular Genetics]", 6th edition, Georg Thieme Verlag, Stuttgart, Germany, 1995) or that of Winnacker ("Gene und Klone [Genes and Clones]", VCH Verlagsgesellschaft, Weinheim, Germany, 1990).

Mutations which lead to a change or reduction in the catalytic properties of enzyme proteins are known from the prior art. Examples which may be mentioned are the works of Qiu and Goodman (Journal of Biological Chemistry 272: 8611-8617 (1997)), Yano et al. (Proceedings of the National Academy of Sciences, USA 95, 5511-5515 (1998), Wente and Schachmann (Journal of Biological Chemistry 266, 20833-20839 (1991). Summarizing descriptions can be found in known textbooks of genetics and molecular biology, such as e.g. that by Hagemann ("Allgemeine Genetik ", Gustav Fischer Verlag, Stuttgart, 1986).

Possible mutations are transitions, transversions, insertions and deletions. Depending on the effect of the amino acid exchange on the enzyme activity, "missense mutations" or "nonsense mutations" are referred to. Insertions or deletions of at least one base pair in a gene lead to "frame shift mutations", which lead to incorrect amino acids being incorporated or translation being interrupted prematurely. Deletions of several codons typically lead to a complete loss of the enzyme activity. Instructions on generation of such mutations are prior art and can be found in known textbooks of genetics and molecular biology, such as e.g. the textbook by Knippers ("Molekulare Genetik [Molecular Genetics]", 6th edition, Georg Thieme Verlag, Stuttgart, Germany, 1995), that by Winnacker ("Gene und Klone ", VCH Verlagsgesellschaft, Weinheim, Germany, 1990) or that by Hagemann ("Allgemeine Genetik ", Gustav Fischer Verlag, Stuttgart, 1986).

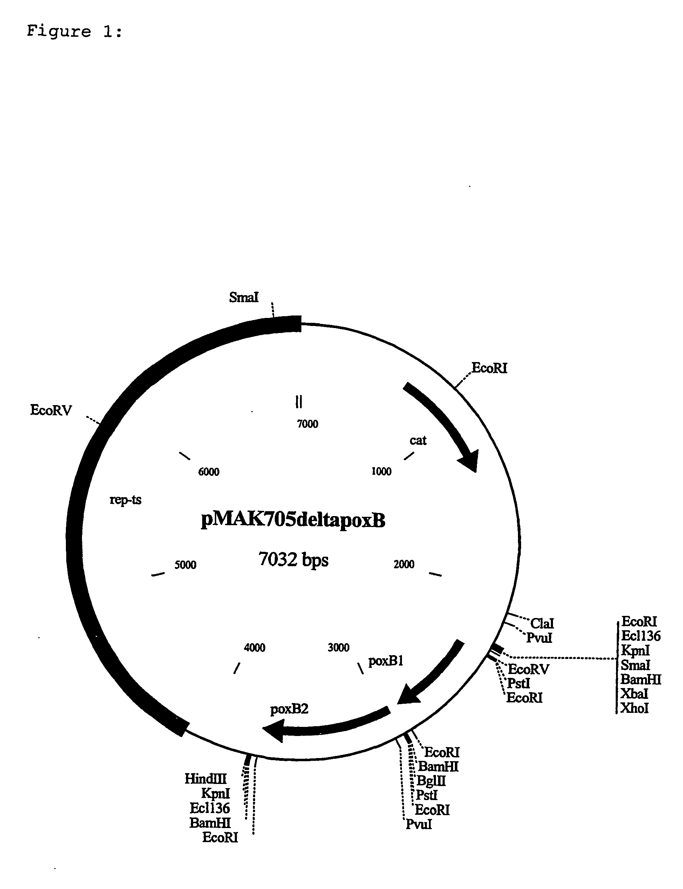

An example of a plasmid with the aid of which the poxB gene of Escherichia coli can be attenuated, in particular eliminated, by position-specific mutagenesis is the plasmid pMAK705ΔpoxB (figure 1). In addition to residues of polylinker sequences, it contains only a part of the 5' and a part of the 3' region of the poxB gene. A 340 bp long section of the coding region is missing (deletion). The sequence of this DNA which can be employed for mutagenesis of the poxB gene is shown in SEQ ID No. 3.

The deletion mutation of the poxB gene can be incorporated into suitable strains by gene or allele replacement.

A conventional method is the method, described by Hamilton et al. (Journal of Bacteriology 174, 4617 - 4622 (1989)), of gene replacement with the aid of a conditionally replicating pSC101 derivative pMAK705. Other methods described in the prior art, such as, for example, those of Martinez-Morales et al. (Journal of Bacteriology 1999, 7143-7148 (1999)) or those of Boyd et al. (Journal of Bacteriology 182, 842-847 (2000)), can likewise be used.

After replacement has taken place, the strain in question contains the form of the ΔpoxB allele shown in SEQ ID No. 4, which is also provided by the invention.

It is also possible to transfer mutations in the poxB gene or mutations which affect expression of the poxB gene into various strains by conjugation or transduction.

It may furthermore be advantageous for the production of the L-amino acids above, in particular L-threonine, with strains of the Enterobacteriaceae family to enhance one or more enzymes of the known threonine biosynthesis pathway or enzymes of anaplerotic metabolism or enzymes for the production of reduced nicotinamide adenine dinucleotide phosphate, in addition to the attenuation of the poxB gene.

The term "enhancement" in this connection describes the increase in the intracellular activity of one or more enzymes or proteins in a microorganism which are coded by the corresponding DNA, for example by increasing the number of copies of the gene or genes, using a potent promoter or a gene which codes for a corresponding enzyme or protein with a high activity, and optionally combining these measures.

Thus, for example, one or more genes chosen from the group consisting of
- the thrABC operon which codes for aspartate kinase, homoserine dehydrogenase, homoserine kinase and threonine synthase (US-A-4,278,765),
- the pyc gene which codes for pyruvate carboxylase (DE-A-19 831 609),
- the pps gene which codes for phosphoenol pyruvate synthase (Molecular and General Genetics 231:332 1992)),
- the ppc gene which codes for phosphoenol pyruvate carboxylase (Gene 31:279-283 (1984)),
- the pntA and pntB genes which code for transhydrogenase (European Journal of Biochemistry 158:647-653 (1986)),
- the rhtB gene which imparts homoserine resistance (EP-A-0 994 190),
- the mqo gene which codes for malate:quinone oxidoreductase (DE 100 348 33.5),
- the rhtC gene which imparts threonine resistance (EP-A-1 013 765), and
- the thrE gene of Corynebacterium glutamicum which codes for threonine export (DE 100 264 94.8) and
- the gdhA gene which codes for glutamate dehydrogenase (Nucleic Acids Research 11: 5257-5266 (1983); Gene 23: 199-209 (1983))
can be enhanced, in particular over-expressed, at the same time.

It may furthermore be advantageous for the production of the L-amino acids above, in particular threonine, in addition to the attenuation of the poxB gene, for one or more genes chosen from the group consisting of
- the tdh gene which codes for threonine dehydrogenase (Ravnikar and Somerville, Journal of Bacteriology 169, 4716-4721 (1987)),
- the mdh gene which codes for malate dehydrogenase (E.C. 1.1.1.37) (Vogel et al., Archives in Microbiology 149, 36-42 (1987)),
- the gene product of the open reading frame (orf) yjfA (Accession Number AAC77180 of the National Center for Biotechnology Information (NCBI, Bethesda, MD, USA),
- the gene product of the open reading frame (orf) ytfP (Accession Number AAC77179 of the National Center for Biotechnology Information (NCBI, Bethesda, MD, USA) and
- the pckA gene which codes for the enzyme phosphoenol pyruvate carboxykinase (Medina et al. (Journal of Bacteriology 172, 7151-7156 (1990))
to be attenuated, in particular eliminated or reduced in expression.

In addition to attenuation of the poxB gene it may furthermore be advantageous for the production of L-amino acids, in particular L-threonine, to eliminate undesirable side reactions (Nakayama: "Breeding of Amino Acid Producing Microorganisms", in: Overproduction of Microbial Products, Krumphanzl, Sikyta, Vanek (eds.), Academic Press, London, UK, 1982).

The microorganisms produced according to the invention can be cultured in the batch process (batch culture), the fed batch (feed process) or the repeated fed batch process (feed process). A summary of known culture methods is described in the textbook by Chmiel (Bioprozesstechnik 1. Einführung in die Bioverfahrenstechnik [Bioprocess Technology 1. Introduction to Bioprocess Technology (Gustav Fischer Verlag, Stuttgart, 1991)) or in the textbook by Storhas (Bioreaktoren und periphere Einrichtungen (Vieweg Verlag, Braunschweig/Wiesbaden, 1994)).

The culture medium to be used must meet the requirements of the particular strains in a suitable manner. Descriptions of culture media for various microorganisms are contained in the handbook "Manual of Methods for General Bacteriology" of the American Society for Bacteriology (Washington D.C., USA, 1981).

Sugars and carbohydrates, such as e.g. glucose, sucrose, lactose, fructose, maltose, molasses, starch and optionally cellulose, oils and fats, such as e.g. soya oil, sunflower oil, groundnut oil and coconut fat, fatty acids, such as e.g. palmitic acid, stearic acid and linoleic acid, alcohols, such as e.g. glycerol and ethanol, and organic acids, such as e.g. acetic acid, can be used as the source of carbon. These substances can be used individually or as a mixture.

Organic nitrogen-containing compounds, such as peptones, yeast extract, meat extract, malt extract, corn steep liquor, soya bean flour and urea, or inorganic compounds, such as ammonium sulfate, ammonium chloride, ammonium phosphate, ammonium carbonate and ammonium nitrate, can be used as the source of nitrogen. The sources of nitrogen can be used individually or as a mixture.

Phosphoric acid, potassium dihydrogen phosphate or dipotassium hydrogen phosphate or the corresponding sodium-containing salts can be used as the source of phosphorus. The culture medium must furthermore comprise salts of metals, such as e.g. magnesium sulfate or iron sulfate, which are necessary for growth. Finally, essential growth substances, such as amino acids and vitamins, can be employed in addition to the abovementioned substances. Suitable precursors can moreover be added to the culture medium. The starting substances mentioned can be added to the culture in the form of a single batch, or can be fed in during the culture in a suitable manner.

Basic compounds, such as sodium hydroxide, potassium hydroxide, ammonia or aqueous ammonia, or acid compounds, such as phosphoric acid or sulfuric acid, can be employed in a suitable manner to control the pH. Antifoams, such as e.g. fatty acid polyglycol esters, can be employed to control the development of foam. Suitable substances having a selective action, e.g. antibiotics, can be added to the medium to maintain the stability of plasmids. To maintain aerobic conditions, oxygen or oxygen-containing gas mixtures, such as e.g. air, are introduced into the culture. The temperature of the culture is usually 25°C to 45°C, and preferably 30°C to 40°C. Culturing is continued until a maximum of L-amino acids or L-threonine has formed. This target is usually reached within 10 hours to 160 hours.

The analysis of L-amino acids can be carried out by anion exchange chromatography with subsequent ninhydrin derivatization, as described by Spackman et al. (Analytical Chemistry, 30, (1958), 1190), or it can take place by reversed phase HPLC as described by Lindroth et al. (Analytical Chemistry (1979) 51:. 1167-1174).

A pure culture of the Escherichia coli K-12 strain DH5α/pMAK705 was deposited as DSM 13720 on 8th September 2000 at the Deutsche Sammlung für Mikroorganismen und Zellkulturen (DSMZ = German Collection of Microorganisms and Cell Cultures, Braunschweig, Germany) in accordance with the Budapest Treaty.

A pure culture of the Escherichia coli K-12 strain MG442ΔpoxB was deposited as DSM 13762 on 2nd October 2000 at the Deutsche Sammlung für Mikroorganismen und Zellkulturen (DSMZ = German Collection of Microorganisms and Cell Cultures, Braunschweig, Germany) in accordance with the Budapest Treaty.

The process according to the invention is used for the fermentative preparation of the L-amino acids L-threonine, L-valine or L-lysine, in particular L-threonine.

The present invention is explained in more detail in the following with the aid of embodiment examples.

The isolation of plasmid DNA from Escherichia coli and all techniques of restriction, Klenow and alkaline phosphatase treatment are carried out by the method of Sambrook et al. (Molecular cloning - A laboratory manual (1989) Cold Spring Harbour Laboratory Press). Unless described otherwise, the transformation of Escherichia coli is carried out by the method of Chung et al. (Proceedings of the National Academy of Sciences of the United States of America USA (1989) 86: 2172-2175).

The incubation temperature for the preparation of strains and transformants is 37°C. Temperatures of 30°C and 44°C are used in the gene replacement method of Hamilton et. al.

### Example 1

### Construction of the deletion mutation of the poxB gene

Parts of the 5' and 3' region of the poxB gene are amplified from Escherichia coli K12 using the polymerase chain reaction (PCR) and synthetic oligonucleotides. Starting from the nucleotide sequence of the poxB gene in E. coli K12 MG1655 (SEQ ID No. 1), the following PCR primers are synthesized (MWG Biotech, Ebersberg, Germany):
poxB'5'-1: 5' - CTGAACGGTCTTAGTGACAG - 3'
poxB'5'-2: 5' - AGGCCTGGAATAACGCAGCAGTTG - 3'
poxB'3'-1: 5' - CTGCGTGCATTGCTTCCATTG - 3'
poxB'3'-2: 5' - GCCAGTTCGATCACTTCATCAC - 3'

The chromosomal E. coli K12 MG1655 DNA employed for the PCR is isolated according to the manufacturers instructions with "Qiagen Genomic-tips 100/G" (QIAGEN, Hilden, Germany). A DNA fragment approx. 500 base pairs (bp) in size from the 5' region of the poxB gene (called poxB1) and a DNA fragment approx. 750 bp in size from the 3' region of the poxB gene (called poxB2) can be amplified with the specific* primers under standard PCR conditions (Innis et al. (1990) PCR Protocols. A Guide to Methods and Applications, Academic Press) with Taq-DNA polymerase (Gibco-BRL, Eggenstein, Germany). The PCR products are each ligated with the vector pCR2.1TOPO (TOPO TA Cloning Kit, Invitrogen, Groningen, The Netherlands) in accordance with the manufacturers instructions and transformed into the E. coli strain TOP10F'.

Selection of plasmid-carrying cells takes place on LB agar, to which 50 µg/ml ampicillin are added. After isolation of the plasmid DNA, the vector pCR2.1TOPOpoxB1 is cleaved with the restriction enzymes Ec1136II and XbaI and, after separation in 0.8% agarose gel, the poxB1 fragment is isolated with the aid of the QIAquick Gel Extraction Kit (QIAGEN, Hilden, Germany). After isolation of the plasmid DNA the vector pCR2.1TOPOpoxB2 is cleaved with the enzymes EcoRV and XbaI and ligated with the poxB1 fragment isolated. The E. coli strain DH5α is transformed with the ligation batch and plasmid-carrying cells are selected on LB agar, to which 50 µg/ml ampicillin is added. After isolation of the plasmid DNA those plasmids in which the mutagenic DNA sequence shown in SEQ ID No. 3 is cloned are detected by control cleavage with the enzymes HindIII and XbaI. One of the plasmids is called pCR2.1TOPOΔpoxB.

### Example 2

### Construction of the replacement vector pMAK705ΔpoxB

The poxB allele described in Example 1 is isolated from the vector pCR2.1TOPOΔpoxB after restriction with the enzymes HindIII and XbaI and separation in 0.8% agarose gel, and ligated with the plasmid pMAK705 (Hamilton et al. (1989) Journal of Bacteriology 174, 4617 - 4622), which has been digested with the enzymes HindIII and XbaI. The ligation batch is transformed in DH5α and plasmid-carrying cells are selected on LB agar, to which 20 µg/ml chloramphenicol is added. Successful cloning is demonstrated after isolation of the plasmid DNA and cleavage with the enzymes HindIII and XbaI. The replacement vector formed, pMAK705ΔpoxB (= pMAK705deltapoxB), is shown in figure 1.

### Example 3

### Position-specific mutagenesis of the poxB gene in the E. coli strain MG442

The L-threonine-producing E. coli strain MG442 is described in the patent specification US-A- 4,278,765 and deposited as CMIM B-1628 at the Russian National Collection for Industrial Microorganisms (VKPM, Moscow, Russia).

For replacement of the chromosomal poxB gene with the plasmid-coded deletion construct, MG442 is transformed with the plasmid pMAK705ΔpoxB, The gene replacement is carried out by the selection method described by Hamilton et al. (1989) Journal of Bacteriology 174, 4617 - 4622) and is verified by standard PCR methods (Innis et al. (1990) PCR Protocols. A Guide to Methods and Applications, Academic Press) with the following oligonucleotide primers:
poxB'5'-1: 5' - CTGAACGGTCTTAGTGACAG - 3'
poxB'3'-2: 5' - GCCAGTTCGATCACTTCATCAC -3'

The strain obtained is called MG442ΔpoxB.

### Example 4

### Preparation of L-threonine with the strain MG442ΔpoxB

MG442ΔpoxB is multiplied on minimal medium with the following composition: 3.5 g/l Na₂HPO₄*2H₂O, 1.5 g/l KH₂PO₄, 1 g/l NH₄Cl, 0.1 g/l MgSO₄*7H₂ 2 g/l glucose, 20 g/l agar. The formation of L-threonine is checked in batch cultures of 10 ml contained in 100 ml conical flasks. For this, 10 ml of preculture medium of the following composition: 2 g/l yeast extract, 10 g/l (NH₄) ₂SO₄, 1 g/l KH₂PO₄, 0.5 g/l MgSO₄*7H₂O, 15 g/l CaCO₃, 20 g/l glucose are inoculated and the batch is incubated for 16 hours at 37°C and 180 rpm on an ESR incubator from Kühner AG (Birsfelden, Switzerland). 250 µl of this preculture are transinoculated into 10 ml of production medium (25 g/l (NH₄) ₂SO₄, 2 g/l KH₂PO₄, 1 g/l MgSO₄*7H₂O, 0.03 g/l FeSO₄*7H₂O, 0.018 g/l MnSO₄*1H₂O 30 g/l CaCO₃, 20 g/l glucose) and the batch is incubated for 48 hours at 37°C. After the incubation the optical density (OD) of the culture suspension is determined with an LP2W photometer from Dr. Lange (Berlin, Germany) at a measurement wavelength of 660 nm.

The concentration of L-threonine formed is then determined in the sterile-filtered culture supernatant with an amino acid analyzer from Eppendorf-BioTronik (Hamburg, Germany) by ion exchange chromatography and post-column reaction with ninhydrin detection.

The result of the experiment is shown in Table 1.

**Table 1**

| Strain | OD (660 nm) | L-Threonine g/l |
|---|---|---|
| MG442 | 6.0 | 1.5 |
| MG442ΔpoxB | 4.9 | 2.6 |

### Example 5

### Preparation of L-threonine with the strain MG442ΔpoxB/pMW218gdhA

### 5.1 Amplification and cloning of the gdhA gene

The glutamate dehydrogenase gene from Escherichia coli K12 is amplified using the polymerase chain reaction (PCR) and synthetic oligonucleotides. Starting from the nucleotide sequence for the gdhA gene in E. coli K12 MG1655 (gene library: Accession No. AE000270 and No. AE000271), PCR primers are synthesized (MWG Biotech, Ebersberg, Germany):
Gdh1: 5' - TGAACACTTCTGGCGGTACG - 3'
Gdh2: 5' - CCTCGGCGAAGCTAATATGG - 3'

The chromosomal E. coli K12 MG1655 DNA employed for the PCR is isolated according to the manufacturers instructions with "QIAGEN Genomic-tips 100/G" (QIAGEN, Hilden, Germany). A DNA fragment approx. 2150 bp in size, which comprises the gdhA coding region and approx. 350 bp 5'-flanking and approx. 450 bp 3'-flanking sequences, can be amplified with the specific primers under standard PCR conditions (Innis et al.: PCR protocols. A guide to methods and applications, 1990, Academic Press) with Pfu-DNA polymerase (Promega Corporation, Madison, USA). The PCR product is cloned in the plasmid pCR2.1TOPO and transformed in the E. coli strain TOP10 (Invitrogen, Leek, The Netherlands, Product Description TOPO TA Cloning Kit, Cat. No. K4500-01). Successful cloning is demonstrated by cleavage of the plasmid pCR2.1TOPOgdhA with the restriction enzymes EcoRI and EcoRV. For this, the plasmid DNA is isolated by means of the "QIAprep Spin Plasmid Kit" (QIAGEN, Hilden, Germany) and, after cleavage, separated in a 0.8% agarose gel.

### 5.2 Cloning of the gdhA gene in the plasmid vector pMW218

The plasmid pCR2.1TOPOgdhA is cleaved with the enzyme EcoRI, the cleavage batch is separated on 0.8% agarose gel and the gdhA fragment 2.1 kbp in size is isolated with the aid of the "OIAquick Gel Extraction Kit" (QIAGEN, Hilden, Germany). The plasmid pMW218 (Nippon Gene, Toyama, Japan) is cleaved with the enzyme EcoRI and ligated with the gdhA fragment. The E. coli strain DH5α is transformed with the ligation batch and pMW218-carrying cells are selected by plating out on LB agar (Lennox, Virology 1955, 1: 190), to which 20µg/ml kanamycin are added.

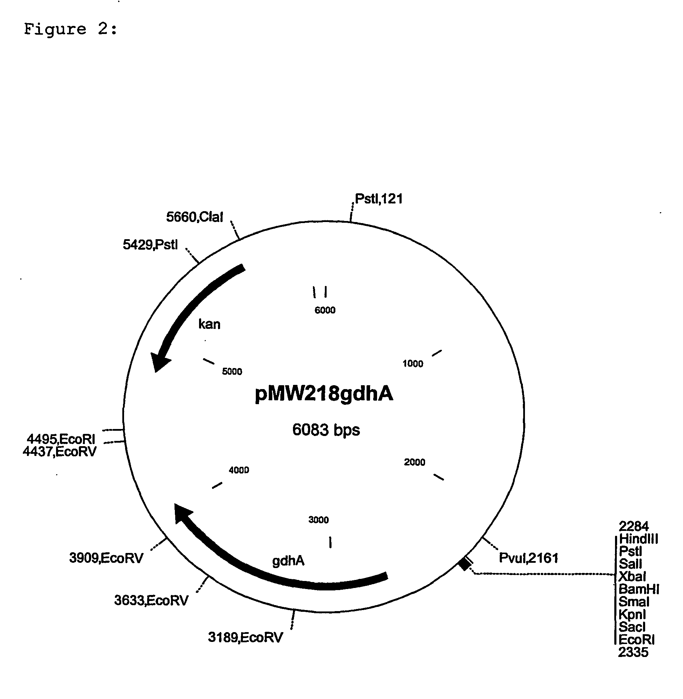

Successful cloning of the gdhA gene can be demonstrated after plasmid DNA isolation and control cleavage with EcoRI and EcoRV. The plasmid is called pMW218gdhA (figure 2).

### 5.3 Preparation of the strain MG442ΔpoxB/pMW218gdhA

The strain MG442ΔpoxB obtained in Example 3 and the strain MG442 are transformed with the plasmid pMW218gdhA and transformants are selected on LB agar, which is supplemented with 20 µg/ml kanamycin. The strains MG442ΔpoxB/pMW218gdhA and MG442/pMW218gdhA are formed in this manner.

### 5.4 Preparation of L-threonine

The preparation of L-threonine by the strains MG442ΔpoxB/pMW218gdhA and MG442/pMW218gdhA is tested as described in Example 4. The minimal medium and the preculture medium are additionally supplemented with 20 µg/ml kanamycin for these two strains.

The result of the experiment is summarized in Table 2.

**Table 2**

| Strain | OD (660 nm) | L-Threonine g/l |
|---|---|---|
| MG442 | 6.0 | 1.5 |
| MG442ΔpoxB | 4.9 | 2.6 |
| MG442/pMW218gdhA | 5.6 | 2.6 |
| MG442ΔpoxB/pMW218gdhA | 5.5 | 2.9 |

### Example 6

### Preparation of L-threonine with the strain MG442ΔpoxB/pMW219rhtC

### 6.1 Amplification of the rhtC gene

The rhtC gene from Escherichia coli K12 is amplified using the polymerase chain reaction (PCR) and synthetic oligonucleotides. Starting from the nucleotide sequence for the rhtC gene in E. coli K12 MG1655 (gene library: Accession No. AE000458, Zakataeva et al. (FEBS Letters 452, 228-232 (1999)), PCR primers are synthesized (MWG Biotech, Ebersberg, Germany):
RhtC1: 5' - CTGTTAGCATCGGCGAGGCA - 3'
RhtC2: 5'.- GCATGTTGATGGCGATGACG - 3'

The chromosomal E. coli K12 MG1655 DNA employed for the PCR is isolated according to the manufacturers instructions with "QIAGEN Genomic-tips 100/G" (QIAGEN, Hilden, Germany). A DNA fragment approx. 800 bp in size can be amplified with the specific primers under standard PCR conditions (Innis et al.: PCR protocols. A guide to methods and applications, 1990, Academic Press) with Pfu-DNA polymerase (Promega Corporation, Madison, USA).

### 6.2 Cloning of the rhtC gene in the plasmid vector pMW219

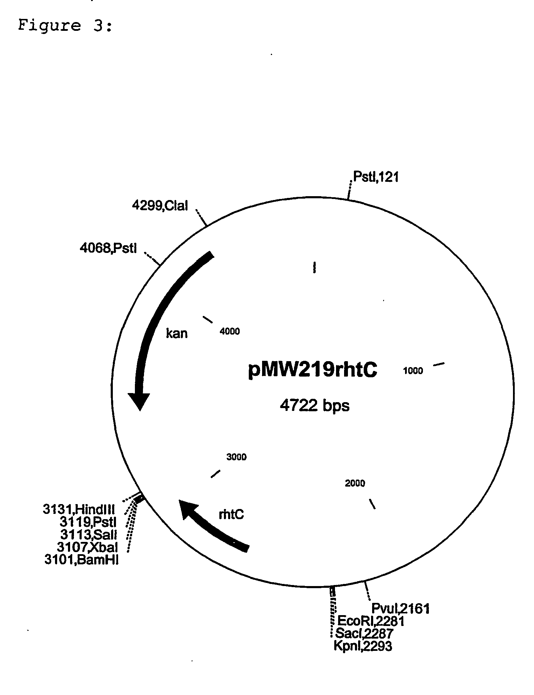

The plasmid pMW219 (Nippon Gene, Toyama, Japan) is cleaved with the enzyme SamI and ligated with the rhtC-PCR fragment. The E. coli strain DH5α is transformed with the ligation batch and pMW219-carrying cells are selected on LB agar, which is supplemented with 20 µg/ml kanamycin. Successful cloning can be demonstrated after plasmid DNA isolation and control cleavage with KpnI, HindIII and NcoI. The plasmid pMW219rhtC is shown in figure 3.

### 6.3 Preparation of the strain MG442ΔpoxB/pMW219rhtC

### The strain MG442ΔpoxB obtained in Example 3 and the strain MG442 are transformed with the plasmid pMW219rhtC and transformants are selected on LB agar, which is supplemented with 20 µg/ml kanamycin. The strains MG442ΔpoxB/pMW219rhtC and MG442/pMW219rhtC are formed in this way.

### 6.4 Preparation of L-threonine

The preparation of L-threonine by the strains MG442ΔpoxB/pMW219rhtC and MG442/pMW219rhtC is tested as described in Example 4. The minimal medium and the preculture medium are additionally supplemented with 20 µg/ml kanamycin for these two strains.

The result of the experiment is summarized in Table 3.

**Table 3**

| Strain | OD (660 nm) | L-Threonine g/l |
|---|---|---|
| MG442 | 6.0 | 1.5 |
| MG442ΔpoxB | 4.9 | 2.6 |
| MG442/pMW219rhtC | 5.2 | 2.9 |
| MG442ΔpoxB/pMW219rhtC | 5.4 | 3.9 |

### Example 7

### Position-specific mutagenesis of the poxB gene in the E. coli strain TOC21R

The L-lysine-producing E. coli strain pDA1/TOC21R is described in the patent application F-A-2511032 and deposited at the Collection Nationale de Culture de Microorganisme (CNCM = National Microorganism Culture Collection, Pasteur Institute, Paris, France) under number I-167. The strain and the plasmid-free host are also described by Dauce-Le Reverend et al. (European Journal of Applied Microbiology and Biotechnology 15:227-231 (1982)) under the name TOCR21/pDA1.

After culture in antibiotic-free LB medium for approximately six generations, a derivative of strain pDA1/TOC21R which no longer contains the plasmid pDA1 is isolated. The strain formed is tetracycline-sensitive and is called TOC21R.

For replacement of the chromosomal poxB gene with the plasmid-coded deletion construct, TOC21R is transformed with the plasmid pMAK705ΔpoxB (Example 2). The gene replacement is carried out by the selection method described by Hamilton et al. (1989) Journal of Bacteriology 174, 4617 - 4622) and is verified by standard PCR methods (Innis et al. (1990) PCR Protocols. A Guide to Methods and Applications, Academic Press) with the following oligonucleotide primers:
poxB'5'-1: 5' - CTGAACGGTCTTAGTGACAG - 3'
poxB'3'-2: 5' - GCCAGTTCGATCACTTCATCAC -3'

The strain obtained is called TOC21RΔpoxB.

### Example 8

### Preparation of L-lysine with the strain TOC21RΔpoxB

The formation of L-lysine by the strains TOC21RΔpoxB and TOC21R is checked in batch cultures of 10 ml contained in 100 ml conical flasks. For this, 10 ml of preculture medium of the following composition: 2 g/l yeast extract, 10 g/l (NH₄)₂SO₄, 1 g/l KH₂PO₄, 0.5 g/l MgSO₄*7H₂ 15 g/l CaCO₃, 20 g/l glucose are inoculated and the batch is incubated for 16 hours at 37°C and 180 rpm on an ESR incubator from Kühner AG (Birsfelden, Switzerland). 250 µl of this preculture are transinoculated into 10 ml of production medium (25 g/l (NH₄)₂SO₄, 2 g/l KH₂PO₄, 1 g/l MgSO₄*7H₂O, 0.03 g/l FeSO₄*7H₂O, 0.018 g/l MnSO₄*1H₂O, 30 g/l CaCO₃, 20 g/l glucose, 25 mg/l L-isoleucine and 5 mg/l thiamine) and the batch is incubated for 72 hours at 37°C. After the incubation the optical density (OD) of the culture suspension is determined with an LP2W photometer from Dr. Lange (Berlin, Germany) at a measurement wavelength of 660 nm.

The concentration of L-lysine formed is then determined in the sterile-filtered culture supernatant with an amino acid analyzer from Eppendorf-BioTronik (Hamburg, Germany) by ion exchange chromatography and post-column reaction with ninhydrin detection.

The result of the experiment is shown in table 4.

**Table 4**

| Strain | OD (660 nm) | L-Lysine g/l |
|---|---|---|
| TOC21R | 1.0 | 1.17 |
| TOC21RΔpoxB | 1.0 | 1.29 |

### Example 9

### Position-specific mutagenesis of the poxB gene in the E. coli strain B-1288

The L-valine-producing E. coli strain AJ 11502 is described in the patent specification US-A-4391907 and deposited at the National Center for Agricultural Utilization Research (Peoria, Illinois, USA) as NRRL B-12288.

After culture in antibiotic-free LB medium for approximately six generations, a plasmid-free derivative of strain AJ 11502 is isolated. The strain formed is ampicillin-sensitive and is called AJ11502kur.

For replacement of the chromosomal poxB gene with the plasmid-coded deletion construct, AJ11502kur is transformed with the plasmid pMAK705ΔpoxB (see Example 2). The gene replacement is carried out by the selection method described by Hamilton et al. (1989) Journal of Bacteriology 174, 4617 - 4622) and is verified by standard PCR methods (Innis et al. (1990) PCR Protocols. A Guide to Methods and Applications, Academic Press) with the following oligonucleotide primers:
poxB'5'-1: 5' - CTGAACGGTCTTAGTGACAG - 3'
poxB'3'-2: 5' - GCCAGTTCGATCACTTCATCAC -3'

The strain obtained is called AJ11502kurΔpoxB. The plasmid described in the patent specification US-A-4391907, which carries the genetic information in respect of valine production, is isolated from strain NRRL B-12288. The strain AJ11502kurΔpoxB is transformed with this plasmid. One of the transformants obtained is called B-12288ΔpoxB.

### Example 10

### Preparation of L-valine with the strain B-12288ΔpoxB

The formation of L-valine by the strains B-12288ΔpoxB and NRRL B-12288 is checked in batch cultures of 10 ml contained in 100 ml conical flasks. For this, 10 ml of preculture medium of the following composition: 2 g/l yeast extract, 10 g/l (NH₄)₂SO₄, 1 g/l KH₂PO₄, 0.5 g/l MgSO₄*7H₂O, 15 g/l CaCO₃, 20 g/l glucose and 50 mg/l ampicillin are inoculated and the batch is incubated for 16 hours at 37°C and 180 rpm on an ESR incubator from Kühner AG (Birsfelden, Switzerland). 250 µl of this preculture are transinoculated into 10 ml of production medium (25 g/l (NH₄)₂SO₄, 2 g/l KH₂PO₄, 1 g/l MgSO₄*7H₂O, 0.03 g/l FeSO₄*7H₂O 0.018 g/l MnSO₄*1H₂O, 30 g/l CaCO₃, 20 g/l glucose, 5 mg/l thiamine and 50 mg/l ampicillin) and the batch is incubated for 72 hours at 37°C. After the incubation the optical density (OD) of the culture suspension is determined with an LP2W photometer from Dr. Lange (Berlin, Germany) at a measurement wavelength of 660 nm.

The concentration of L-valine formed is then determined in the sterile-filtered culture supernatant with an amino acid analyzer from Eppendorf-BioTronik (Hamburg, Germany) by ion exchange chromatography and post-column reaction with ninhydrin detection.

The result of the experiment is shown in table 5.

**Table 5**

| Strain | OD (660 nm) | L-Valine g/l |
|---|---|---|
| NRRL B-12288 | 5.7 | 0.95 |
| B-12288ΔpoxB | 5.6 | 1.05 |

### Brief Description of the Figures

- Figure 1: pMAK705ΔpoxB (= pMAK705deltapoxB)
- Figure 2: pMW218gdhA
- Figure 3: pMW219rhtC

The length data are to be understood as approx. data. The abbreviations and designations used have the following meaning:

| | | |
|---|---|---|
| • | cat: | chloramphenicol resistance gene |
| | rep-ts: | temperature-sensitive replication region of the plasmid pSC101 |
| | poxB1: | part of the 5' region of the poxB gene |
| | poxB2: | part of the 3' region of the poxB gene |
| | kan: | kanamycin resistance gene |
| | gdhA: | glutamate dehdyrogensase gene |
| | rhtC: | gene imparting threonine resistance |

The abbreviations for the restriction enzymes have the following meaning

| | | |
|---|---|---|
| • | BamHI: | restriction endonuclease from Bacillus amyloliquefaciens |
| • | BglII: | restriction endonuclease from Bacillus globigii |
| • | ClaI: | restriction endonuclease from Caryphanon latum |
| | Ec1136II | restriction endonuclease from Enterobacter cloacae RFL136 (= Ecl136) |
| • | EcoRI: | restriction endonuclease from Escherichia coli |
| • | EcoRV: | restriction endonuclease from Escherichia coli |
| • | HindIII: | restriction endonuclease from Haemophilus influenzae |
| • | KpnI: | restriction endonuclease from Klebsiella pneumoniae |
| • | PstI: | restriction endonuclease from Providencia stuartii |
| • | PvuI: | restriction endonuclease from Proteus vulgaris |
| • | SacI; | restriction endonuclease from Streptomyces achromogenes |
| • | SalI: | restriction endonuclease from Streptomyces albus |
| • | SmaI: | restriction endonuclease from Serratia marcescens |
| • | XbaI: | restriction endonuclease from Xanthomonas badrii |
| | XhoI: | restriction endonuclease from Xanthomonas holcicola |

## Claims

1. A process for the fermentative preparation of L-threonine, L-valine or L-lysine, which comprises carrying out the following steps:
a) fermentation of the microorganisms of the Enterobacteriaceae family which produce the desired L-amino acid and in which at least the poxB gene or nucleotide sequences which code for its gene product are attenuated, in particular eliminated,
b) concentration of the L-amino acid in the medium or in the cells of the bacteria and
c) isolation of the L-amino acid.

2. A process as claimed in claim 1, which comprises employing microorganisms in which further genes of the biosynthesis pathway of the desired L-amino acid are additionally enhanced.

3. A process as claimed in claim 1, which comprises employing microorganisms in which the metabolic pathways which reduce the formation of the desired L-amino acid are at least partly eliminated.

4. A process as claimed in claim 1, which comprises attenuating, in particular eliminating, expression of the polynucleotide(s) which code(s) for the poxB gene product.

5. A process as claimed in claim 1, which comprises reducing the regulatory and/or catalytic properties of the polypeptide (enzyme protein) for which the polynucleotide poxB codes.

6. A process as claimed in claim 1, which comprises fermenting, for the preparation of L-threonine, microorganisms of the Enterobacteriaceae family in which one or more genes chosen from the group consisting of:
6.1 the thrABC operon which codes for aspartate kinase, homoserine dehydrogenase, homoserine kinase and threonine synthase,
6.2 the pyc gene which codes for pyruvate carboxylase,
6.3 the pps gene which codes for phosphoenol pyruvate synthase,
6.4 the ppc gene which codes for phosphoenol pyruvate carboxylase,
6.5 the pntA and pntB genes which code for transhydrogenase,
6.6 the rhtB gene which imparts homoserine resistance,
6.7 the mqo gene which codes for malate:quinone oxidoreductase,
6.8 the rhtC gene which imparts threonine resistance,
6.9 the thrE gene which codes for threonine export and
6.10 the gdhA gene which codes for glutamate dehydrogenase is or are enhanced, in particular over-expressed, at the same time.

7. A process as claimed in claim 1, which comprises fermenting, for the preparation of L-threonine, microorganisms of the Enterobacteriaceae family in which one or more genes chosen from the group consisting of:
7.1 the tdh gene which codes for threonine dehydrogenase,
7.2 the mdh gene which codes for malate dehydrogenase,
7.3 the gene product of the open reading frame (orf) yjfA,
7.4 the gene product of the open reading frame (orf) ytfP, and
7.5 the pckA gene which codes for the enzyme phosphoenol pyruvate carboxykinase,
is or are attenuated, in particular eliminated or reduced in expression, at the same time.

8. A microorganism of the Enterobacteriaceae family which produces L-threonine, in which the poxB gene or nucleotide sequences which code for its gene product are attenuated, in particular eliminated, and which have a resistance to α-amino-β-hydroxyvaleric acid and optionally a compensatable partial need for L-isoleucine.

9. The Escherichia coli K-12 strain MG442ΔpoxB deposited at the Deutsche Sammlung für Mikroorganismen und Zellkulturen (DSMZ = German Collection of Microorganisms and Cell Cultures, Braunschweig, Germany) under no. DSM 13762.

10. An isolated polynucleotide from microorganisms of the Enterobacteriaceae family, containing the polynucleotide sequence of SEQ ID No. 4, in particular suitable as a constituent of plasmids for position-specific mutagenesis of the poxB gene.

11. A strain of the Enterobacteriaceae family which produces L-threonine and contains SEQ ID No. 4.

## Patentansprüche

1. Verfahren zur fermentativen Herstellung von L-Threonin, L-Valin oder L-Lysin, bei dem man folgende Schritte durchführt:
a) Fermentation der die gewünschte L-Aminosäure produzierenden Mikroorganismen der Familie Enterobacteriaceae, in denen man zumindest das poxB-Gen oder für dessen Genprodukt kodierende Nukleotidsequenzen abschwächt, insbesondere ausschaltet,
b) Anreicherung der L-Aminosäure im Medium oder in den Zellen der Bakterien und
c) Isolieren der L-Aminosäure.

2. Verfahren nach Anspruch 1, bei dem man Mikroorganismen einsetzt, in denen man zusätzlich weitere Gene des Biosyntheseweges der gewünschten L-Aminosäure verstärkt.

3. Verfahren nach Anspruch 1, bei dem man Mikroorganismen einsetzt, in denen die Stoffwechselwege zumindest teilweise ausgeschaltet sind, die die Bildung der gewünschten L-Aminosäure verringern.

4. Verfahren nach Anspruch 1, bei dem man die Expression des (der) Polynukleotide(s), das (die) für das poxB-Genprodukt kodiert (kodieren), abschwächt, insbesondere ausschaltet.

5. Verfahren nach Anspruch 1, bei dem man die regulatorischen und/oder katalytischen Eigenschaften des Polypeptids (Enzymproteins) verringert, für das das Polynukleotid poxB kodiert.

6. Verfahren nach Anspruch 1, bei dem man zur Herstellung von L-Threonin Mikroorganismen der Familie Enterobacteriaceae fermentiert, in denen man gleichzeitig eines oder mehrere der Gene, ausgewählt aus der Gruppe:
6.1 das für die Aspartatkinase, die Homoserin-Dehydrogenase, die Homoserinkinase und die Threoninsynthase kodierende thrABC-Operon,
6.2 das für die Pyruvat-Carboxylase kodierende pyc-Gen,
6.3 das für die Phosphoenolpyruvat-Synthase kodierende pps-Gen,
6.4 das für die Phosphoenolpyruvat-Carboxylase kodierende ppc-Gen,
6.5 die für die Transhydrogenase kodierenden Gene pntA und pntB,
6.6 das Homoserinresistenz vermittelnde Gen rhtB,
6.7 das für die Malat:Chinon-Oxidoreductase kodierende mqo-Gen,
6.8 das Threoninresistenz vermittelnde Gen rhtC,
6.9 das für den Threoninexport kodierende thrE-Gen und
6.10 das für die Glutamat-Dehydrogenase kodierende gdhA-Gen,
verstärkt, insbesondere überexprimiert.

7. Verfahren nach Anspruch 1, bei dem man zur Herstellung von L-Threonin Mikroorganismen der Familie Enterobacteriaceae fermentiert, in denen man gleichzeitig eines oder mehrere der Gene, ausgewählt aus der Gruppe:
7.1 das für die Threonin-Dehydrogenase kodierende tdh-Gen,
7.2 das für die Malat-Dehydrogenase kodierende mdh-Gen,
7.3 das Genprodukt des offenen Leserahmens (orf) yjfA,
7.4 das Genprodukt des offenen Leserahmens (orf) ytfP und
7.5 das für die Enzym-Phosphoenolpyruvat-Carboxykinase kodierende pckA-Gen,
abschwächt, insbesondere ausschaltet oder die Expression verringert.

8. L-Threonin produzierende Mikroorganismen der Familie Enterobacteriaceae, in denen man das poxB-Gen oder für dessen Genprodukt kodierende Nukleotidsequenzen abschwächt, insbesondere ausschaltet und die eine Resistenz gegen α-Amino-βhydroxyvaleriansäure und gegebenenfalls eine kompensierbare partielle Bedürftigkeit für L-Isoleucin besitzen.

9. Escherichia coli K-12 Stamm MG442ΔpoxB, hinterlegt unter der Nummer DSM 13762 bei der Deutschen Sammlung für Mikroorganismen und Zellkulturen (DSMZ, Braunschweig, Deutschland).

10. Isoliertes Polynukleotid aus Mikroorganismen der Familie Enterobacteriaceae, enthaltend die Polynukleotidsequenz der SEQ ID No. 4, insbesondere geeignet als Bestandteil von Plasmiden für die ortsspezifische Mutagenase des poxB-Gens.

11. L-Threonin produzierende Stämme der Familie Enterobacteriaceae, enthaltend SEQ ID No. 4.

## Revendications

1. Processus pour la préparation fermentative de L-thréonine, de L-valine ou de L-lysine, dans lequel les étapes suivantes sont effectuées :
a) une fermentation des microorganismes de la famille des Entérobactériacés produisant l'acide aminé L souhaité et dans lesquels au moins le gène poxB ou des séquences nucléotidiques codant pour son produit du gène est (sont) atténué(es), en particulier éliminé(es) ;
b) une concentration de l'acide aminé L dans le milieu ou dans les cellules des bactéries ; et
c) un isolement de l'acide aminé L.

2. Processus selon la revendication 1, dans lequel des microorganismes sont utilisés, dans lesquels d'autres gènes de la voie de biosynthèse de l'acide aminé L souhaité sont amplifiés en plus.

3. Processus selon la revendication 1, dans lequel des microorganismes sont utilisés, dans lesquels les voies métaboliques qui réduisent la formation de l'acide aminé L souhaité sont eliminées au moins partiellement.

4. Processus selon la revendication 1, dans lequel l'expression du (des) polynucléotide(s) codant pour le produit du gène poxB est atténuée, en particulier eliminée.

5. Processus selon la revendication 1, dans lequel les propriétés de régulation et/ou catalytiques du polypeptide (protéine enzymatique) codé par le polynucléotide poxB sont réduites.

6. Processus selon la revendication 1, dans lequel, pour la préparation de L-thréonine, des microorganismes de la famille des Entérobactériacés sont fermentés, dans lesquels un ou plusieurs gène(s) choisi(s) dans le groupe comprenant :
6.1 l'opéron thrABC codant pour une aspartate kinase, une homosérine déshydrogénase, une homosérine kinase et une thréonine synthase, 6.2 le gène pyc codant pour une pyruvate carboxylase,
6.3 le gène pps codant pour une phosphoénol pyruvate synthase,
6.4 le gène ppc codant pour une phosphoénol pyruvate carboxylase,
6.5 les gènes pntA et pntB codant pour une transhydrogénase,
6.6 le gène rhtB qui confère une résistance à l'homosérine,
6.7 le gène mqo codant pour une malate : quinone oxidoréductase,
6.8 le gène rhtC qui confère une résistance à la thréonine,
6.9 le gène thrE codant pour un export de thréonine, et
6.10 le gène gdhA codant pour une glutamate déshydrogénase,
est ou sont amplifiés en particulier surexprimés simultanément.

7. Processus selon la revendication 1, dans lequel, pour la préparation de L-thréonine, des microorganismes de la famille des Entérobactériacés sont fermentés, dans lesquels un ou plusieurs gène(s) choisi(s) dans le groupe comprenant :
7.1 le gène tdh codant pour une thréonine déshydrogénase,
7.2 le gène mdh codant pour une malate déshydrogénase,
7.3 le produit génique du cadre ouvert de lecture (orf) yjfA,
7.4 le produit génique du cadre ouvert de lecture (orf) ytfP et
7.5 le gène pckA codant pour l'enzyme phosphoénol pyruvate carboxykinase,
est ou sont atténués, en particulier leur expression est éliminée ou réduite simultanément.

8. Microorganisme, de la famille des Entérobactériacés productrice de L-thréonine, dans lequel le gène poxB ou des séquences nucléotidiques codant pour son produit de gène sont attenués, en particulier eliminés, et qui ont une résistance à l'acide α-amino-β-hydroxyvalérique et éventuellement un besoin partiel en L-isoleucine compensable.

9. Souche MG442ΔpoxB d'*Escherichia coli* K-12 déposée sous le numéro DSM 13762 auprès du Deutsche Sammlung für Mikroorganismen und Zellkulturen (DSMZ = Collection allemande de microorganismes et de cultures de cellules, Braunschweig, Allemagne).

10. Polynucléotide isolé à partir de microorganismes de la famille des Entérobactériacés contenant la séquence de polynucléotides de la SEQ ID n° : 4, qui est particulièrement approprié comme constituant de plasmides destinés à une mutagenèse du gène poxB spécifique d'une position.

11. Souche de la famille des Entérobactériacés productrice de L-thréonine qui contient la SEQ ID n° : 4.


### SEQUENCE PROTOCOL

<110> Degussa AG
<120> Process for the fermentative preparation of L-amino acids using strains of the Enterobacteriaceae family.
<130> 000613 BT
<140>
   <141>
<160> 4
<170> PatentIn Ver. 2.1
<210> 1
   <211> 1719
   <212> DNA
   <213> Escherichia coli
<220>
   <221> CDS
   <222> (1)..(1716)
   <223> poxB
<400> 1
   atg aaa caa acg gtt gca gct tat atc gcc aaa aca ctc gaa tcg gca    48
   Met Lys Gln Thr Val Ala Ala Tyr Ile Ala Lys Thr Leu Glu Ser Ala
    1               5                  10                  15
   ggg gtg aaa cgc atc tgg gga gtc aca ggc gac tct ctg aac ggt ctt    96
   Gly Val Lys Arg Ile Trp Gly Val Thr Gly Asp Ser Leu Asn Gly Leu
               20                  25                  30
   agt gac agt ctt aat cgc atg ggc acc atc gag tgg atg tcc acc cgc   144
   Ser Asp Ser Leu Asn Arg Met Gly Thr Ile Glu Trp Met Ser Thr Arg
           35                  40                  45
   cac gaa gaa gtg gcg gcc ttt gcc gct ggc gct gaa gca caa ctt agc   192
   His Glu Glu Val Ala Ala Phe Ala Ala Gly Ala Glu Ala Gln Leu Ser
       50                  55                  60
   gga gaa ctg gcg gtc tgc gcc gga tcg tgc ggc ccc ggc aac ctg cac   240
   Gly Glu Leu Ala Val Cys Ala Gly Ser Cys Gly Pro Gly Asn Leu His
   65                  70                  75                  80
   tta atc aac ggc ctg ttc gat tgc cac cgc aat cac gtt ccg gta ctg   288
   Leu Ile Asn Gly Leu Phe Asp Cys His Arg Asn His Val Pro Val Leu
                   85                  90                  95
   gcg att gcc gct cat att ccc tcc agc gaa att ggc agc ggc tat ttc   336
   Ala Ile Ala Ala His Ile Pro Ser Ser Glu Ile Gly Ser Gly Tyr Phe
               100                 105                 110
   cag gaa acc cac cca caa gag cta ttc cgc gaa tgt agt cac tat tgc   384
   Gln Glu Thr His Pro Gln Glu Leu Phe Arg Glu Cys Ser His Tyr Cys
           115                 120                 125
   gag ctg gtt tcc agc ccg gag cag atc cca caa gta ctg gcg att gcc    432
   Glu Leu Val Ser Ser Pro Glu Gln Ile Pro Gln Val Leu Ala Ile Ala
       130                 135                 140
   atg cgc aaa gcg gtg ctt aac cgt ggc gtt tcg gtt gtc gtg tta cca    480
   Met Arg Lys Ala Val Leu Asn Arg Gly Val Ser Val Val Val Leu Pro
   145                 150                 155                 160
   ggc gac gtg gcg tta aaa cct gcg cca gaa ggg gca acc atg cac tgg    528
   Gly Asp Val Ala Leu Lys Pro Ala Pro Glu Gly Ala Thr Met His Trp
                   165                 170                 175
   tat cat gcg cca caa cca gtc gtg acg ccg gaa gaa gaa gag tta cgc    576
   Tyr His Ala Pro Gln Pro Val Val Thr Pro Glu Glu Glu Glu Leu Arg
               180                 185                 190
   aaa ctg gcg caa ctg ctg cgt tat tcc agc aat atc gcc ctg atg tgt    624
   Lys Leu Ala Gln Leu Leu Arg Tyr Ser Ser Asn Ile Ala Leu Met Cys
           195                 200                 205
   ggc agc ggc tgc gcg ggg gcg cat aaa gag tta gtt gag ttt gcc ggg    672
   Gly Ser Gly Cys Ala Gly Ala His Lys Glu Leu Val Glu Phe Ala Gly
       210                 215                 220
   aaa att aaa gcg cct att gtt cat gcc ctg cgc ggt aaa gaa cat gtc    720
   Lys Ile Lys Ala Pro Ile Val His Ala Leu Arg Gly Lys Glu His Val
   225                 230                 235                 240
   gaa tac gat aat ccg tat gat gtt gga atg acc ggg tta atc ggc ttc    768
   Glu Tyr Asp Asn Pro Tyr Asp Val Gly Met Thr Gly Leu Ile Gly Phe
                   245                 250                 255
   tcg tca ggt ttc cat acc atg atg aac gcc gac acg tta gtg cta ctc    816
   Ser Ser Gly Phe His Thr Met Met Asn Ala Asp Thr Leu Val Leu Leu
               260                 265                 270
   ggc acg caa ttt ccc tac cgc gcc ttc tac ccg acc gat gcc aaa atc    864
   Gly Thr Gln Phe Pro Tyr Arg Ala Phe Tyr Pro Thr Asp Ala Lys Ile
           275                 280                 285
   att cag att gat atc aac cca gcc agc atc ggc gct cac agc aag gtg    912
   Ile Gln Ile Asp Ile Asn Pro Ala Ser Ile Gly Ala His Ser Lys Val
       290                 295                 300
   gat atg gca ctg gtc ggc gat atc aag tcg act ctg cgt gca ttg ctt    960
   Asp Met Ala Leu Val Gly Asp Ile Lys Ser Thr Leu Arg Ala Leu Leu
   305                 310                 315                 320
   cca ttg gtg gaa gaa aaa gcc gat cgc aag ttt ctg gat aaa gcg ctg    1008
   Pro Leu Val Glu Glu Lys Ala Asp Arg Lys Phe Leu Asp Lys Ala Leu
                   325                 330                 335
   gaa gat tac cgc gac gcc cgc aaa ggg ctg gac gat tta gct aaa ccg    1056
   Glu Asp Tyr Arg Asp Ala Arg Lys Gly Leu Asp Asp Leu Ala Lys Pro
               340                 345                 350
   agc gag aaa gcc att cac ccg caa tat ctg gcg cag caa att agt cat    1104
   Ser Glu Lys Ala Ile His Pro Gln Tyr Leu Ala Gln Gln Ile Ser His
           355                 360                 365
   ttt gcc gcc gat gac gct att ttc acc tgt gac gtt ggt acg cca acg     1152
   Phe Ala Ala Asp Asp Ala Ile Phe Thr Cys Asp Val Gly Thr Pro Thr
       370                 375                 380
   gtg tgg gcg gca cgt tat cta aaa atg aac ggc aag cgt cgc ctg tta     1200
   Val Trp Ala Ala Arg Tyr Leu Lys Met Asn Gly Lys Arg Arg Leu Leu
   385                 390                 395                 400
   ggt tcg ttt aac cac ggt tcg atg gct aac gcc atg ccg cag gcg ctg     1248
   Gly Ser Phe Asn His Gly Ser Met Ala Asn Ala Met Pro Gln Ala Leu
                   405                 410                 415
   ggt gcg cag gcg aca gag cca gaa cgt cag gtg gtc gcc atg tgc ggc     1296
   Gly Ala Gln Ala Thr Glu Pro Glu Arg Gln Val Val Ala Met Cys Gly
               420                 425                 430
   gat ggc ggt ttt agc atg ttg atg ggc gat ttc ctc tca gta gtg cag     1344
   Asp Gly Gly Phe Ser Met Leu Met Gly Asp Phe Leu Ser Val Val Gln
           435                 440                 445
   atg aaa ctg cca gtg aaa att gtc gtc ttt aac aac agc gtg ctg ggc     1392
   Met Lys Leu Pro Val Lys Ile Val Val Phe Asn Asn Ser Val Leu Gly
       450                 455                 460
   ttt gtg gcg atg gag atg aaa gct ggt ggc tat ttg act gac ggc acc     1440
   Phe Val Ala Met Glu Met Lys Ala Gly Gly Tyr Leu Thr Asp Gly Thr
   465                 470                 475                 480
   gaa cta cac gac aca aac ttt gcc cgc att gcc gaa gcg tgc ggc att     1488
   Glu Leu His Asp Thr Asn Phe Ala Arg Ile Ala Glu Ala Cys Gly Ile
                   485                 490                 495
   acg ggt atc cgt gta gaa aaa gcg tct gaa gtt gat gaa gcc ctg caa     1536
   Thr Gly Ile Arg Val Glu Lys Ala Ser Glu Val Asp Glu Ala Leu Gln
               500                 505                 510
   cgc gcc ttc tcc atc gac ggt ccg gtg ttg gtg gat gtg gtg gtc gcc     1584
   Arg Ala Phe Ser Ile Asp Gly Pro Val Leu Val Asp Val Val Val Ala
           515                 520                 525
   aaa gaa gag tta gcc att cca ccg cag atc aaa ctc gaa cag gcc aaa     1632
   Lys Glu Glu Leu Ala Ile Pro Pro Gln Ile Lys Leu Glu Gln Ala Lys
       530                 535                 540
   ggt ttc agc ctg tat atg ctg cgc gca atc atc agc gga cgc ggt gat     1680
   Gly Phe Ser Leu Tyr Met Leu Arg Ala Ile Ile Ser Gly Arg Gly Asp
   545                 550                 555                 560
   gaa gtg atc gaa ctg gcg aaa aca aac tgg cta agg taa                 1719
   Glu Val Ile Glu Leu Ala Lys Thr Asn Trp Leu Arg
                   565                 570
<210> 2
   <211> 572
   <212> PRT
   <213> Escherichia coli
<400> 2
   Met Lys Gln Thr Val Ala Ala Tyr Ile Ala Lys Thr Leu Glu Ser Ala
     1               5                  10                  15
   Gly Val Lys Arg Ile Trp Gly Val Thr Gly Asp Ser Leu Asn Gly Leu
               20                       25                       30
   Ser Asp Ser Leu Asn Arg Met Gly Thr Ile Glu Trp Met Ser Thr Arg
           35                  40                  45
   His Glu Glu Val Ala Ala Phe Ala Ala Gly Ala Glu Ala Gln Leu Ser
       50                  55                  60
   Gly Glu Leu Ala Val Cys Ala Gly Ser Cys Gly Pro Gly Asn Leu His
   65                  70                  75                  80
   Leu Ile Asn Gly Leu Phe Asp Cys His Arg Asn His Val Pro Val Leu
                   85                  90                  95
   Ala Ile Ala Ala His Ile Pro Ser Ser Glu Ile Gly Ser Gly Tyr Phe
               100                 105                 110
   Gln Glu Thr His Pro Gln Glu Leu Phe Arg Glu Cys Ser His Tyr Cys
           115                 120                 125
   Glu Leu Val Ser Ser Pro Glu Gln Ile Pro Gln Val Leu Ala Ile Ala
       130                 135                 140
   Met Arg Lys Ala Val Leu Asn Arg Gly Val Ser Val Val Val Leu Pro
   145                 150                 155                 160
   Gly Asp Val Ala Leu Lys Pro Ala Pro Glu Gly Ala Thr Met His Trp
                   165                 170                 175
   Tyr His Ala Pro Gln Pro Val Val Thr Pro Glu Glu Glu Glu Leu Arg
               180                 185                 190
   Lys Leu Ala Gln Leu Leu Arg Tyr Ser Ser Asn Ile Ala Leu Met Cys
           195                 200                 205
   Gly Ser Gly Cys Ala Gly Ala His Lys Glu Leu Val Glu Phe Ala Gly
       210                 215                 220
   Lys Ile Lys Ala Pro Ile Val His Ala Leu Arg Gly Lys Glu His Val
   225                 230                 235                 240
   Glu Tyr Asp Asn Pro Tyr Asp Val Gly Met Thr Gly Leu Ile Gly Phe
                   245                 250                 255
   Ser Ser Gly Phe His Thr Met Met Asn Ala Asp Thr Leu Val Leu Leu
               260                 265                 270
   Gly Thr Gln Phe Pro Tyr Arg Ala Phe Tyr Pro Thr Asp Ala Lys Ile
           275                 280                 285
   Ile Gln Ile Asp Ile Asn Pro Ala Ser Ile Gly Ala His Ser Lys Val
       290                 295                 300
   Asp Met Ala Leu Val Gly Asp Ile Lys Ser Thr Leu Arg Ala Leu Leu
   305                 310                 315                 320
   Pro Leu Val Glu Glu Lys Ala Asp Arg Lys Phe Leu Asp Lys Ala Leu
                   325                 330                 335
   Glu Asp Tyr Arg Asp Ala Arg Lys Gly Leu Asp Asp Leu Ala Lys Pro
               340                 345                 350
   Ser Glu Lys Ala Ile His Pro Gln Tyr Leu Ala Gln Gln Ile Ser His
                   355                 360                 365
   Phe Ala Ala Asp Asp Ala Ile Phe Thr Cys Asp Val Gly Thr Pro Thr
       370                 375                 380
   Val Trp Ala Ala Arg Tyr Leu Lys Met Asn Gly Lys Arg Arg Leu Leu
   385                 390                 395                 400
   Gly Ser Phe Asn His Gly Ser Met Ala Asn Ala Met Pro Gln Ala Leu
                   405                 410                 415
   Gly Ala Gln Ala Thr Glu Pro Glu Arg Gln Val Val Ala Met Cys Gly
               420                 425                 430
   Asp Gly Gly Phe Ser Met Leu Met Gly Asp Phe Leu Ser Val Val Gln
           435                 440                 445
   Met Lys Leu Pro Val Lys Ile Val Val Phe Asn Asn Ser Val Leu Gly
       450                 455                 460
   Phe Val Ala Met Glu Met Lys Ala Gly Gly Tyr Leu Thr Asp Gly Thr
   465                 470                 475                 480
   Glu Leu His Asp Thr Asn Phe Ala Arg Ile Ala Glu Ala Cys Gly Ile
                   485                 490                 495
   Thr Gly Ile Arg Val Glu Lys Ala Ser Glu Val Asp Glu Ala Leu Gln
               500                 505                 510
   Arg Ala Phe Ser Ile Asp Gly Pro Val Leu Val Asp Val Val Val Ala
           515                 520                 525
   Lys Glu Glu Leu Ala Ile Pro Pro Gln Ile Lys Leu Glu Gln Ala Lys
       530                 535                 540
   Gly Phe Ser Leu Tyr Met Leu Arg Ala Ile Ile Ser Gly Arg Gly Asp
   545                 550                 555                 560
   Glu Val Ile Glu Leu Ala Lys Thr Asn Trp Leu Arg
                   565                 570
<210> 3
   <211> 1454
   <212> DNA
   <213> Escherichia coli
<220>
   <221> misc_feature
   <222> (1)..(1454)
   <223> Mutagenic DNA
<220>
   <221> misc_feature
   <222> (1)..(56)
   <223> Technical DNA/residues of the polylinker sequence
<220>
   <221> misc_feature
   <222> (57) .. (577)
   <223> Part of the 5' region (poxB1) of the poxB gene
<220>
   <221> misc feature
   <222> (578) .. (646)
   <223> Technical DNA/residues of the polylinker sequence
<220>
   <221> misc feature
   <222> (647)..(1398)
   <223> Part of the 3' region (poxB2) of the poxB gene
<220>
   <221> misc feature
   <222> (1399)..(1454)
   <223> Technical DNA/residues of the polylinker sequence
<400> 3
   ctagatgcat gctcgagcgg ccgccagtgt gatggatatc tgcagaattc gcccttctga 60
   acggtcttag tgacagtctt aatcgcatgg gcaccatcga gtggatgtcc acccgccacg 120
   aagaagtggc ggcctttgcc gctggcgctg aagcacaact tagcggagaa ctggcggtct 180
   gcgccggatc gtgcggcccc ggcaacctgc acttaatcaa cggcctgttc gattgccacc 240
   gcaatcacgt tccggtactg gcgattgccg ctcatattcc ctccagcgaa attggcagcg 300
   gctatttcca ggaaacccac ccacaagagc tattccgcga atgtagtcac tattgcgagc 360
   tggtttccag cccggagcag atcccacaag tactggcgat tgccatgcgc aaagcggtgc 420
   ttaaccgtgg cgtttcggtt gtcgtgttac caggcgacgt ggcgttaaaa cctgcgccag 480
   aaggggcaac catgcactgg tatcatgcgc cacaaccagt cgtgacgccg gaagaagaag 540
   agttacgcaa actggcgcaa ctgctgcgtt attccaggcc taagggcgaa ttccagcaca 600
   ctggcggccg ttactagtgg atccgagatc tgcagaattc gcccttctgc gtgcattgct 660
   tccattggtg gaagaaaaag ccgatcgcaa gtttctggat aaagcgctgg aagattaccg 720
   cgacgcccgc aaagggctgg acgatttagc taaaccgagc gagaaagcca ttcacccgca 780
   atatctggcg cagcaaatta gtcattttgc cgccgatgac gctattttca cctgtgacgt 840
   tggtacgcca acggtgtggg cggcacgtta tctaaaaatg aacggcaagc gtcgcctgtt 900
   aggttcgttt aaccacggtt cgatggctaa cgccatgccg caggcgctgg gtgcgcaggc 960
   gacagagcca gaacgtcagg tggtcgccat gtgcggcgat ggcggtttta gcatgttgat 1020
   gggcgatttc ctctcagtag tgcagatgaa actgccagtg aaaattgtcg tctttaacaa 1080
   cagcgtgctg ggctttgtgg cgatggagat gaaagctggt ggctatttga ctgacggcac 1140
   cgaactacac gacacaaact ttgcccgcat tgccgaagcg tgcggcatta cgggtatccg 1200
   tgtagaaaaa gcgtctgaag ttgatgaagc cctgcaacgc gccttctcca tcgacggtcc 1260
   ggtgttggtg gatgtggtgg tcgccaaaga agagttagcc attccaccgc agatcaaact 1320
   cgaacaggcc aaaggtttca gcctgtatat gctgcgcgca atcatcagcg gacgcggtga 1380
   tgaagtgatc gaactggcaa gggcgaattc cagcacactg gcggccgtta ctagtggatc 1440
   cgagctcggt acca                                                   1454
<210> 4
   <211> 1448
   <212> DNA
   <213> Escherichia coli
<220>
   <221> misc_feature
   <222> (1)..(3)
   <223> Start codon of the delta poxB allele
<220>
   <221> misc_feature
   <222> (1)..(605)
   <223> 5' region of the delta poxB allele
<220>
   <221> misc_feature
   <222> (606)..(674)
   <223> Technical DNA/residues of the polylinker sequence
<220>
   <221> misc feature
   <222> (675)..(1445)
   <223> 3' region of the delta poxB allele
<220>
   <221> misc_feature
   <222> (1446)..(1448)
   <223> Stop codon of the delta poxB allele
<400> 4
   atgaaacaaa cggttgcagc ttatatcgcc aaaacactcg aatcggcagg ggtgaaacgc 60
   atctggggag tcacaggcga ctctctgaac ggtcttagtg acagtcttaa tcgcatgggc 120
   accatcgagt ggatgtccac ccgccacgaa gaagtggcgg cctttgccgc tggcgctgaa 180
   gcacaactta gcggagaact ggcggtctgc gccggatcgt gcggccccgg caacctgcac 240
   ttaatcaacg gcctgttcga ttgccaccgc aatcacgttc cggtactggc gattgccgct 300
   catattccct ccagcgaaat tggcagcggc tatttccagg aaacccaccc acaagagcta 360
   ttccgcgaat gtagtcacta ttgcgagctg gtttccagcc cggagcagat cccacaagta 420
   ctggcgattg ccatgcgcaa agcggtgctt aaccgtggcg tttcggttgt cgtgttacca 480
   ggcgacgtgg cgttaaaacc tgcgccagaa ggggcaacca tgcactggta tcatgcgcca 540
   caaccagtcg tgacgccgga agaagaagag ttacgcaaac tggcgcaact gctgcgttat 600
   tccaggccta agggcgaatt ccagcacact ggcggccgtt actagtggat ccgagatctg 660
   cagaattcgc ccttctgcgt gcattgcttc cattggtgga agaaaaagcc gatcgcaagt 720
   ttctggataa agcgctggaa gattaccgcg acgcccgcaa agggctggac gatttagcta 780
   aaccgagcga gaaagccatt cacccgcaat atctggcgca gcaaattagt cattttgccg 840
   ccgatgacgc tattttcacc tgtgacgttg gtacgccaac ggtgtgggcg gcacgttatc 900
   taaaaatgaa cggcaagcgt cgcctgttag gttcgtttaa ccacggttcg atggctaacg 960
   ccatgccgca ggcgctgggt gcgcaggcga cagagccaga acgtcaggtg gtcgccatgt 1020
   gcggcgatgg cggttttagc atgttgatgg gcgatttcct ctcagtagtg cagatgaaac 1080
   tgccagtgaa aattgtcgtc tttaacaaca gcgtgctggg ctttgtggcg atggagatga 1140
   aagctggtgg ctatttgact gacggcaccg aactacacga cacaaacttt gcccgcattg 1200
   ccgaagcgtg cggcattacg ggtatccgtg tagaaaaagc gtctgaagtt gatgaagccc 1260
   tgcaacgcgc cttctccatc gacggtccgg tgttggtgga tgtggtggtc gccaaagaag 1320
   agttagccat tccaccgcag atcaaactcg aacaggccaa aggtttcagc ctgtatatgc 1380
   tgcgcgcaat catcagcgga cgcggtgatg aagtgatcga actggcgaaa acaaactggc 1440
   taaggtaa 1448